# EUROPÄISCHE PATENTSCHRIFT

(11) **EP 1 656 914 B1**
(45) Veröffentlichungstag und Bekanntmachung des Hinweises auf die Patenterteilung: **14.07.2010**
(21) Anmeldenummer: 05024518.2
(22) Anmeldetag: 10.11.2005
(51) Int. Cl.: A61F 13/00

(54) **Einweg-Absorptionskörper zum Aufsaugen von aus den Wunden austretendem, flüssigem Exsudat**
One way absorbent for absorbing liquid exudate from wounds
Absorbant de non retour pour absorber les exsudats de blessures

(30) Priorität: 10.11.2004 DE 202004017465 U
(43) Veröffentlichungstag der Anmeldung: 17.05.2006
(73) Patentinhaber: Riesinger, Birgit, 48346 Ostbevern (DE)
(72) Erfinder: Riesinger, Birgit, 48346 Ostbevern (DE)
(74) Vertreter: Michalski Hüttermann & Partner Patentanwälte

(56) Entgegenhaltungen:
- EP-A- 0 311 364
- EP-B- 1 345 636
- WO-A-00/33778
- DE-A1- 4 233 289
- DE-A1- 10 059 439
- DE-A1- 10 309 558
- US-A1- 2002 065 494
- US-A1- 2004 033 270
- US-A1- 2004 121 682
- US-A1- 2004 157 971
- US-B1- 6 436 418

## Beschreibung

Die Erfindung betrifft einen Einweg-Absorptionskörper zum Aufsaugen von aus den Wunden austretendem, flüssigem Exsudat, aufweisend:
- eine flüssigkeitsdurchlässige, aus zwei Seitenwänden bestehende Hülle,
- und wenigstens eine Lage eines flüssigkeitsabsorbierenden Textilabschnittes mit darin vorhandenen Superabsorber-Teilchen, die in der Hülle untergebracht ist,
wobei das absorbierte Exsudat innerhalb des Absorptionskörpers und damit innerhalb der Hülle bis zur Entfernung des Absorptionskörpers von dem Körper des Patienten verbleibt, und wobei der Absorptionskörper sich in der Nähe seiner maximalen Füllung mit Flüssigkeiten in seinem Querschnitt der Kreisform annähert.

Ein Einweg-Absorptionskörper der oben genannten Art ist der DE 100 59 439 A1 der Anmelderin bekannt. Obwohl der bekannte Einweg-Absorptionskörper sich in der Praxis bewährt hat, wird eine Verbesserung seiner wundheilenden Eigenschaften angestrebt.

In der US 6 436 418 B1 wird ein für den Hygienebereich bestimmtes Einwegprodukt offenbart, bestehend aus einer Hülle; einer flüssigkeitsabsorbierenden, mit Superabsorberteilchen durchsetzten Textilabschnitt und aseptisch wirkenden Substanzen. Die Hülle besitzt eine flüssigkeitsundurchlässige Unterseite (backsheet) und eine flüssigkeitsdurchlässige, dem Benutzer zugewandte, Oberseite (topsheet). Die aseptisch wirkende Substanzen, wie z.B. Silberoxid sind in das Material der Hüllenoberseite eingearbeitet oder darauf aufgetragen. Die Verwendung als Einwegprodukt für das Auffangen von z.B. Urin schließt per se eine Verwendung im Wundheilungsbereich aus, da hier sehr viel strengere Anforderungen an die Materialen und deren Ausführung gestellt werden. Darüber hinaus ist der Aufbau mit einer flüssigkeitsundurchlässigen Seite ungeeignet für Einweg-Absorptionskörper bei denen die Durchlässigkeit für das Wundexudat von allen Seiten gewährleistet werden muß.

Aus US 2004/157971 A1 ist ein Superabsorbens mit geruchshemmender Wirkung bekannt, dass jedoch ebenfalls für den Hygienebereich konzipiert ist und nicht in einer Wundauflage verwendet werden kann.

Die hydrophilen, mit Superabsorberteilchen durchsetzten Polyurethanschäume aus DE 42 33 289 A1 können auf ein Trägervlies aufgetragen werden, das dann in der Wundversorgung eingesetzt werden kann. Ein gesonderter Absorptionskörper mit einer antimikrobiellen Substanz zum Einsatz in der Wundheilung wird nicht beschrieben.

Das Gleiche gilt für die in der WO 00/33778 A1 offenbarten bakteriziden, absorbierenden Verbände aus einer superabsorbierenden, polymeren Matrix und einer Menge an bakterizider Substanz, hier quaternäre Ammoniumverbindungen. Ein gesonderter Einweg-Absorptionskörper für die Wundbehandlung fehlt auch hier.

Aufgabe der vorliegenden Erfindung ist es daher einen Einweg-Absorptionskörper zum Aufsaugen von aus der Wunde austretendem, flüssigem Exsudat bereit zu stellen, der durch seine Konstruktion und seine speziellen Eigenschaften verbesserte wundheilende Wirkung besitzt.

Diese Aufgabe ist durch einen gattungsgemäßen Einweg-Absorptionskörper dadurch gelöst, dass dieser eine Menge an einer antimikrobiell wirkenden silberhaltigen Substanz aufweist und in den flüssigkeitsabsorbierenden Textilabschnitt oder die darum befindliche Hülle eingebracht ist.

Die antimikrobiell wirkende Substanz kann in einem zusätzlichen flüssigkeitsdurchlässigen Textilabschnitt enthalten sein, der zusammen mit dem die Superabsorber-Teilchen aufweisenden Textilabschnitt innerhalb der Hülle angeordnet ist.

Bei einer anderen Ausführung kann der Absorptionskörper eine Menge an einer antimikrobiell wirkenden Substanz aufweisen, die in das Material der Hülle eingebettet ist oder an der Hülle haftet.

Die antimikrobiell wirkende Substanz kann auch direkt in den flüssigkeitsaufzunehmenden, die Superabsorber-Teilchen aufweisenden Textilabschnitt eingearbeitet, beispielsweise eingestreut sein.

Vorzugsweise liegt die antimikrobielle Substanz flächenhaft vor.

Bei einer weiteren Ausführungsform kann zwischen der Hülle und einer diese bedeckenden Lage einer antiadhäsiven Perforationsfolie ein zusätzlicher flüssigkeitsdurchlässiger, die antimikrobielle Substanz aufweisender Textilabschnitt angeordnet sein. Hierbei kann die antiadhäsive Folie Perforationen aufweisen, die jeweils trichterförmig nach innen gerichtet sind, wodurch eine verkleinerte Wundauflagefläche gebildet ist. Abgesehen davon kann eine derartige antiadhäsive, die Wundauflagefläche verkleinernde Perforationsfolie am Einweg-Absorptionskörper gemäß den anderen Ausführungsformen eingebracht sein.

Die antimikrobiell wirkende Substanz kann beispielsweise silber-, kupfer- oder zinkhaltig sein. Hierzu können Trägersubstanzen vorgesehen sein, die in das Material der Textilabschnitte, der Hülle oder der zusätzlichen Lage eingebettet oder in sonstiger Weise eingebracht sind. So löst sich die antimikrobielle Substanz beim Aufsaugen von Exsudat nicht oder nur kaum aus dem Absorptionskörper heraus und ist nur in sehr reduziertem Umfang bioverfügbar.

Die silberhaltige Substanz kann aus nanokristallinen atomaren oder elementaren Verbindungen, wie Silberionen, bestehen, die ein breites Wirkungsspektrum aufweisen. Die silberhaltige Substanz kann beispielsweise in Pulver- oder Granulatform vorliegen.

Der zusätzliche Textilabschnitt kann auch eine Menge an Aktivkohle enthalten.

Der Einweg-Absorptionskörper kann Salze oder Ester der Alginsäure enthalten, insbesondere mit Natrium oder Calcium versehene Alginat-Präparate.

Das Material der beiden Textilabschnitte und/oder der Hülle kann aus Mikrofilamenten, beispielsweise aus sogenannten Hydrofasern bestehen. Es kommen auch stark hygroskopische Alginatfasern in Frage.

Die Verwendungsmöglichkeiten der erfindungsgemäßen Einweg-Absorptionskörpers sind nicht nur auf wundheilung begrenzt. Er kann auch zum Aufsaugen von flüssigen Ausscheidungen aus chirurgisch angelegten Organausleitungen am menschlichen oder tierischen Körper zum Einsatz kommen.

Weitere Vorteile des Einweg-Absorptionskörpers gemäß der Erfindung sind den nachfolgenden Ausführungsbeispielen zu entnehmen, die anhand der Zeichnung näher erläutert sind. Die Figuren zeigen:
- Fig. 1: einen Einweg-Absorptionskörper gemäß der Erfindung in einer ersten Ausführungsform, in einer Draufsicht auf seine Flachseite;
- Fig. 2: einen Schnitt A-A gemäß der Fig. 1;
- Fig. 3: einen Einweg-Absorptionskörper gemäß der Erfindung in einer zweiten Ausführungsform, in einem schematischen Schnitt;
- Fig. 4: einen Einweg-Absorptionskörper gemäß der Erfindung in einer dritten Ausführungsform, ebenfalls in einem schematischen Schnitt;
- Fig. 5: einen Einweg-Absorptionskörper gemäß den Figuren 1 und 2, jedoch mit einer Lage einer die Hülle bedeckenden anderen Folie, in einem schematischen Schnitt;
- Fig. 6: einen Einweg-Absorptionskörper gemäß der Erfindung in einer vierten Ausführungsform, ebenso in einem schematischen Schnitt;
- Fig. 7: schematisch einen Abschnitt einer Perforationsfolie;
- Fig. 8: einen Einweg-Absorptionskörper mit einer die Hülle bedeckenden, flüssigkeitsdurchlässigen, rauhen Folie, in einem schematischen Schnitt;
- Fig. 9: einen Einweg-Absorptionskörper mit einer darauf angeklebten Kolloid-Folie, in einer Draufsicht auf seine Flachseite;
- Fig. 10: einen an einer Wunde angeklebten EinwegAbsorptionskörper mit Anschlusstelle zur Luftevakuierung, in einem schematischen Schnitt;
- Fig. 11: einen Einweg-Absorptionskörper mit einer innerhalb der Hülle liegenden, kleineren und den zusätzlichen Textilabschnitt enthaltenden Hülle, ebenfalls in einem schematischen Schnitt.

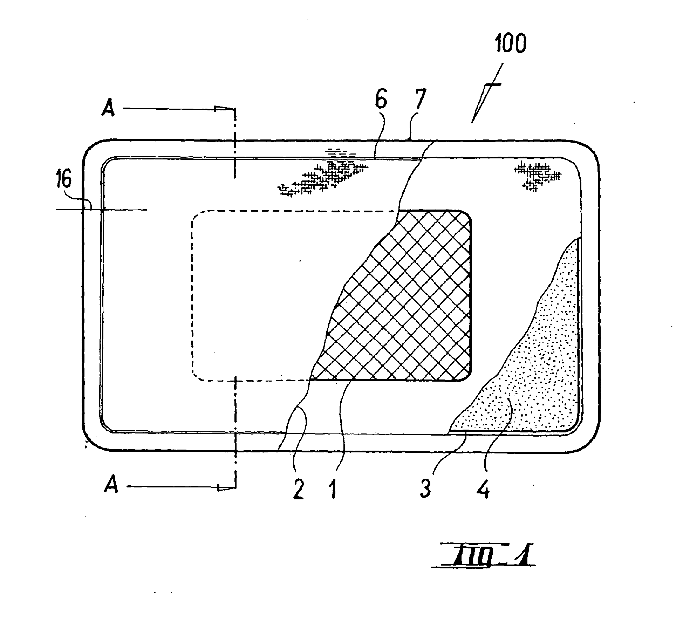

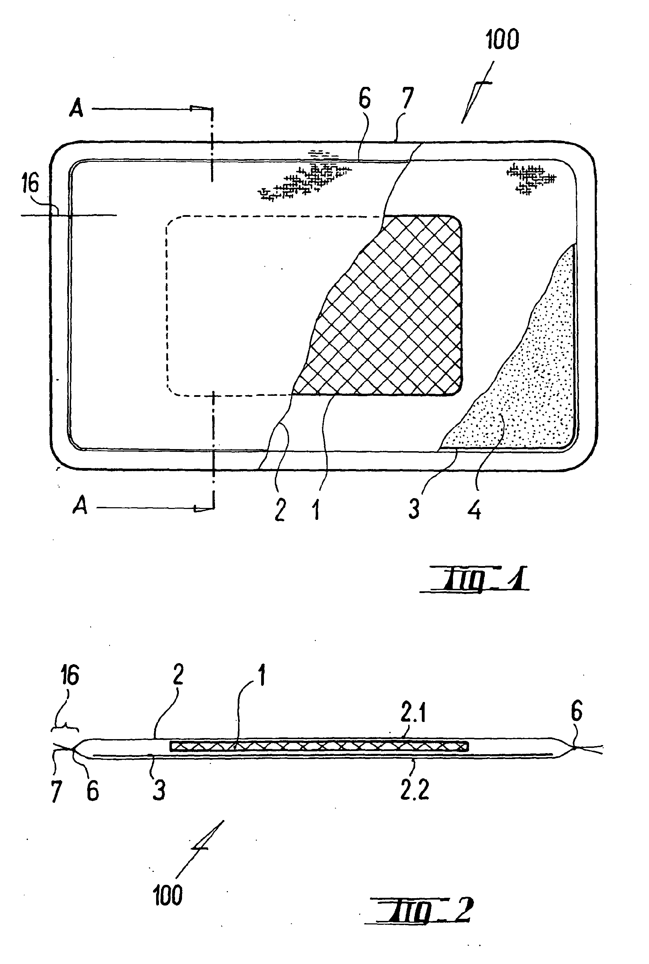

Die Figuren 1 und 2 zeigen einen Einweg-Absorptionskörper 100, der sich aus einer flächenhaften Hülle 2, einem innerhalb der Hülle 2 untergebrachten, mit Superabsorber-Teilchen angereicherten Textilabschnitt 1 und einem zusätzlichen, mit antimikrobiellen Substanzen durchsetzten Textilabschnitt 3 zusammensetzt.

Die Hülle 2 besteht aus zwei Seitenwänden 2.1, 2.2, die miteinander über eine umlaufende Naht 6 verbunden sind. Die Naht 6 ist vorzugsweise im Ultraschallschweißverfahren angefertigt, so dass keine zusätzlichen Garne mehr vorhanden sind, die die Naht versteifen und verdicken können. Von großem Vorteil ist, dass die Hülle 2 einen Überstand 16 aufweist, welcher durch einen umlaufenden Bereich zwischen der Naht 6 und einer freien Außenkante 7 der Hülle 2 definiert ist. Die Seitenwände 2.1, 2.2 sind in dem Bereich miteinander nicht verbunden. Der Überstand 16 stellt also einen wirksamen Schutz vor schmerzhaften Berührungen der Wunde mit der Naht 6 dar. Die Hülle 2 ist aus einer hauchdünnem Perforationsfolie aus Polyethylen gefertigt.

Der flüssigkeitsabsorbierende Textilabschnitt 1 enthält quellbare, Hydrogel bildende Polymerisate, sogenannte Superabsorber (SAP), in Form einer Matrix hydrophiler Teilchen im Textilabschnitt. Diese sind in der Lage, die Exsudate zu absorbieren und zu binden, so dass der Rücktransport der Flüssigkeitsmoleküle nach außen erschwert ist.

Wichtig ist, dass die Lage des flüssigkeitsabsorbierenden Textilabschnittes 1 in Draufsicht auf ihre Flachseite eine Fläche (vgl. Fig. 1) hat, die wesentlich kleiner als die der Hülle 2 ist. Im vorliegenden Fall ist die Fläche des Textilabschnittes 1 etwa 40% kleiner als die Fläche der Hülle 2. Dadurch kann der Textilabschnitt 1 sein Quellvermögen beim Aufsagen völlig entfalten, ohne durch die Seitenwände der Hülle begrenzt zu werden. So kann der Querschnitt des Einweg-Absorptionskörpers 100 beim Aufsaugen nahezu eine Kreisform annehmen, wobei die ausgedehnte Hülle 2 keine bzw. minimale Kräfte auf den aufgequollenen Textilabschnitt 1 ausübt. In dieser Form kann der Einweg-Absorptionskörpers 100, dessen Hülle 2 im wesentlichen trocken bleibt, aus dem Körper des Patienten entfernt und entsorgt werden.

wie die Fig.2 zeigt, liegt der zusätzliche, flüssigkeitsdurchlässige Textilabschnitt 3 zwischen dem flüssigkeitsabsorbierenden Textilabschnitt 1 und der Seitenwand 2.2, mit der der Einweg-Absorptionskörpers 100 auf die Wunde auflegbar ist. Der Textilabschnitt 3 ist mit Aktivkohle und einer antimikrobiell wirkenden, silberhaltigen Substanz 4, hier: nanokristalline Silberionen, durchsetzt. Die silberhaltige Substanz 4 eignet sich, wie bekannt, zur Bekämpfung von multiresistenten Keimen. Die Substanz 4 wurde in Pulverform in den Textilabschnitt 3 eingestreut.

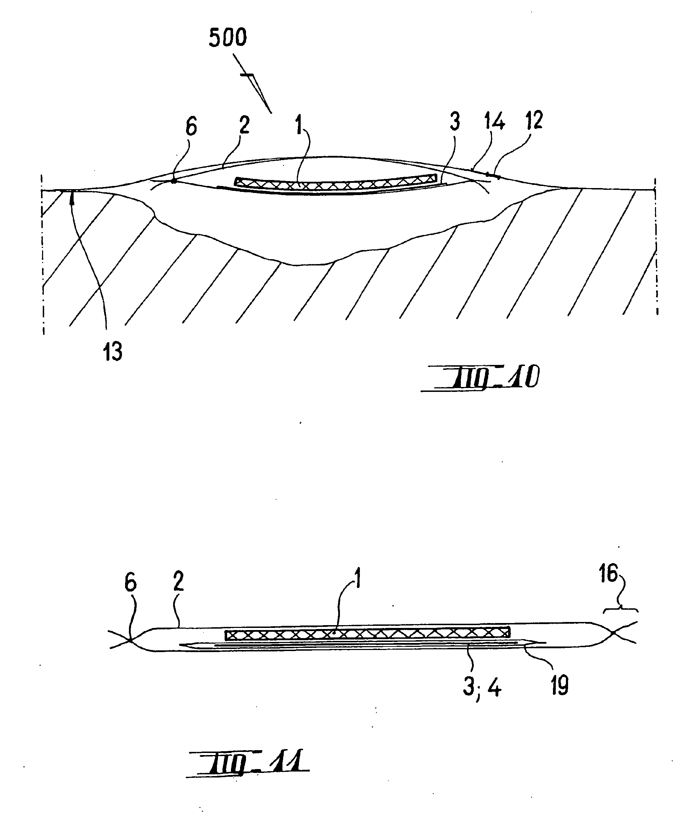

Eine von der oben beschriebenen etwas abweichende Ausführungsform ist der Fig. 11 zu entnehmen. Der Unterschied zu der in den Figuren 1 und 2 dargestellten Ausführungs- form liegt darin, dass der zusätzliche, antimikrobielle Textilabschnitt 3 von einer ebenfalls flüssigkeitsdurchlässigen Hülle 19 umgeben ist.

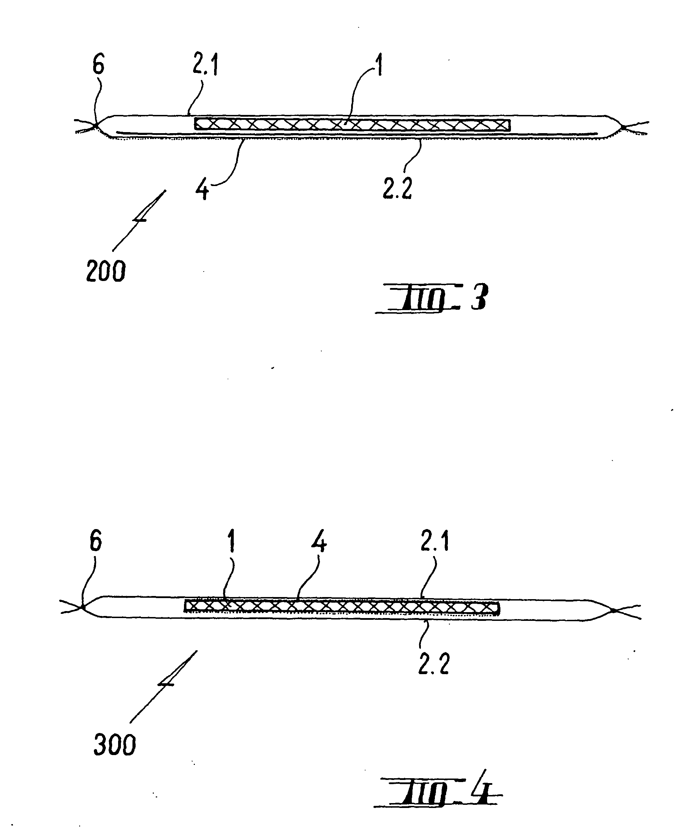

Auf den Textilabschnitt 3 kann verzichtet werden, wenn die silberhaltige Substanz 4 gemäß einer anderen Ausführungsform (Bezugszahl 300; vgl. Fig. 4) direkt in den flüssigkeitsabsorbierenden Textilabschnitt 1 oder auch gemäß einer weiteren Ausführungsform (Bezugszahl 200; vgl. Fig. 3) ins Material der Seitenwand der Hülle 2 eingestreut ist.

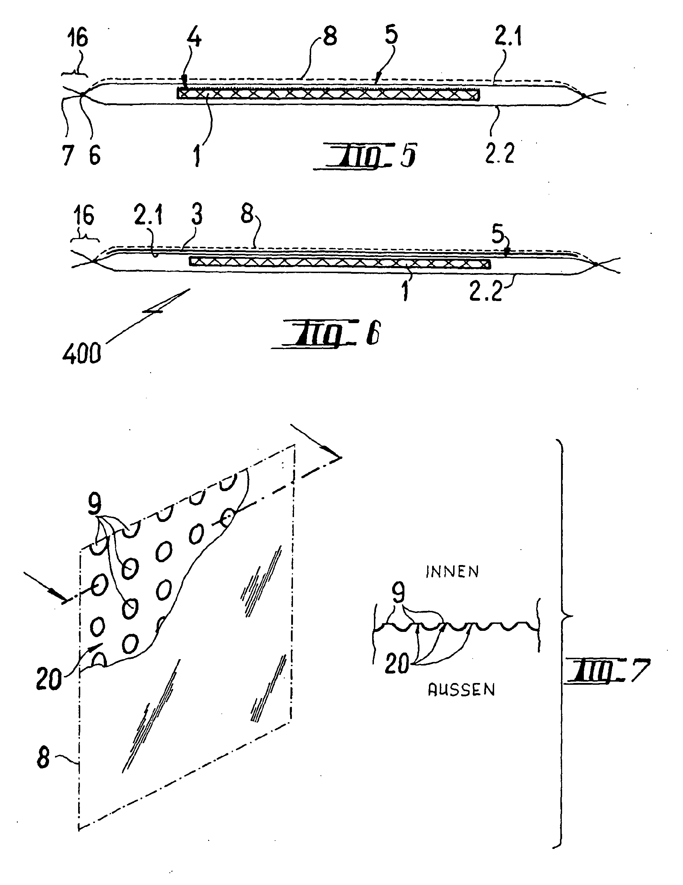

Gemäß den Figuren 5 und 6 ist auf eine Außenfläche 5 der Seitenwand 2.1 oder 2.2 eine Lage 8 einer antiadhäsiven, glatten Perforationsfolie gelegt und mit derselben Naht 6 mit der Hülle verschweißt, so dass der freie Überstand 16 unverändert verbleibt. Die Lage 8 ist nicht mit der Hülle 2 verklebt. Bei der in Fig. 5 dargestellten Ausführungsform ist die antimikrobielle Substanz 4 direkt in den Textilabschnitt 1 eingestreut.

Die Lage 8 der Folie weist Perforationen 9 auf, die jeweils trichterförmig nach innen, d. h. in Richtung Textilabschnitt 1 bzw. der Hülle 2 gerichtet sind. Da jede Perforation in einer Vertiefung liegt, ergibt sich eine verkleinerte Wundauflagefläche 20, die der Fig. 7 zu entnehmen ist.

Die Ausführungsform gemäß der Fig. 6 (Bezugszahl 400) zeigt einen Einweg-Absorptionskörper 400, der mit einer oben beschriebenen Lage 8 der Perforationsfolie versehen ist, wobei zwischen der äußeren Lage 8 und der Seitenwand 2.1 der Hülle 2 der antimikrobiell ausgerüstete Textilabschnitt 3 lose angeordnet ist.

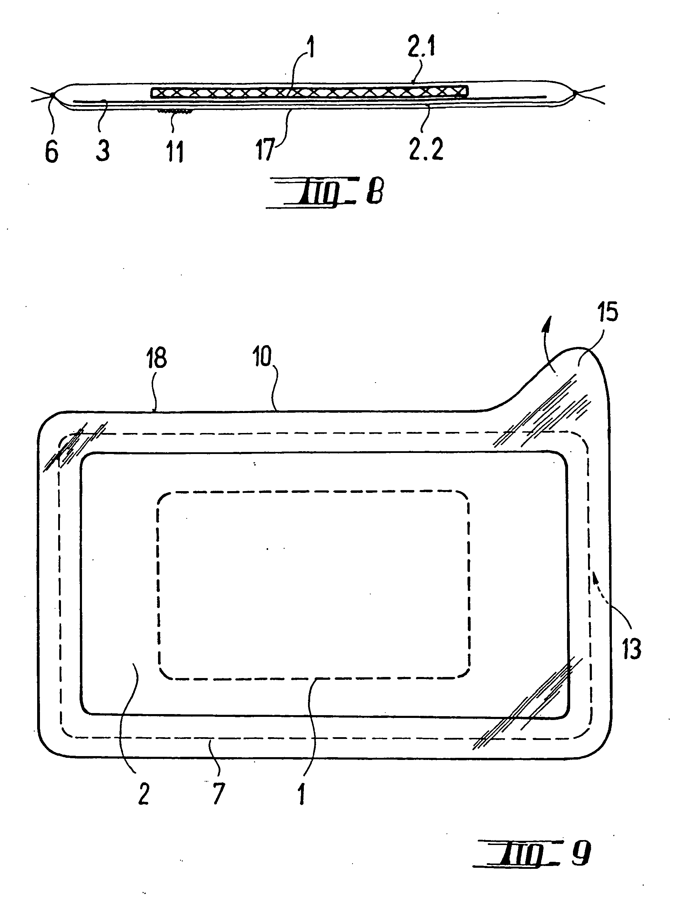

In Anlehnung an die in den Figuren 5 und 6 dargestellten Ausführungsformen wird auch vorgeschlagen, den Einweg-Absorptionskörper zusätzlich mit einer Lage 17 einer flüssigkeitsdurchlässigen Folie von einer rauhen Oberflächenstruktur 11 auszustatten (vgl. Fig. 8). Innerhalb der Hülle 2 eines solchen Einweg-Absorptionskörpers befinden sich beide Textilabschnitte 1; 3, wobei auf die Hülle 2 ist die Lage 17 gelegt und mittels der Ultraschallnaht 6 mit der Hülle peripher verbunden. Die rauhe Oberflächenstruktur 11 ermöglicht es, die Granulation durch eine mechanische Reibung im Wundbereich anzuregen.

Gemäß der Fig. 9 ist auf die Außenfläche 5 der Hülle 2 eine Hydrokolloid-Folie 10 in Form eines Rings 18 aufgeklebt. Der Ring 18 überlappt die Außenkante 7 der Hülle 2 unter Belassung einer umlaufenden Klebefläche 13 zum Ankleben auf die Haut des Patienten. Das bioverträgliche Hydrokolloid-Material bildet einen erwünschten Wundrandschutz. Auf der Unterseite des Absorptionskörpers ist eine abziehbare Schutzfolie 15 angeordnet.

Schließlich ist in der Fig. 10 ein weiterer Einweg-Absorptionskörper 500 gezeigt, der alle Merkmale der in den Figuren 1 und 2 dargestellten ersten Ausführungsform aufweist, wobei ein zusätzliches Element, nämlich ein flüssigkeitsundurchlässiger Folienabschnitt 14 vorhanden ist, an dem eine Anschlußstelle 12 in Form einer Öffnung zur Luftevakuierung aus dem Bereich der Wunde und zum Aufrechterhalten des Unterdrucks eingebracht worden ist. An die Anschlußstelle 12 kann eine Vakuumpumpe, Vakuumflasche oder dgl. über eine nicht dargestellte Schlauchleitung angeschlossen werden. Der Folienabschnitt 14 ist flächenmäßig größer als die Hülle 2 und weist ebenfalls eine Klebefläche 13 zum Ankleben des Absorptionskörpers auf die Haut des Patienten auf. Hierzu eignet sich auch die vorgenannte Hydrokolloid-Folie in Form eines Rings.

Der Folienabschnitt 14 ist transparent, wobei an der anliegenden Seitenwand der Hülle 2 ein transparentes Fenster zur Beobachtung des Wundbereichs eingebracht werden kann (nicht dargestellt).

### Bezugszeichenliste:

- 1: Textilabschnitt
- 2: Hülle
- 2.1; 2.2: Seitenwand (v. 2)
- 3: (zusätzlicher) Textilabschnitt
- 4: (antimikrobielle) Substanz
- 5: Außenfläche
- 6: Naht
- 7: Außenkante
- 8: Lage
- 9: Perforation
- 10: Hydrokolloid-Folie
- 11: Oberflächenstruktur
- 12: Anschlußstelle
- 13: Klebefläche
- 14: Folienabschnitt
- 15: Schutzfolie
- 16: Überstand
- 17: Lage
- 18: Ring
- 19: Hülle (innen)
- 20: Wundauflagefläche

- 100...500: Einweg-Absorptionskörper

## Patentansprüche

1. Einweg-Absorptionskörper (100) zum Aufsaugen von aus der Wunde austretendem, flüssigem Exsudat, aufweisend:
- eine flüssigkeitsdurchlässige, aus zwei Seitenwänden (2.1, 2.2) bestehende Hülle (2),
- und wenigstens eine Lage eines flüssigkeitsabsorbierenden Textilabschnittes (1) mit darin vorhandenen Superabsorber-Teilchen, die in der Hülle (2) untergebracht ist,
wobei das absorbierende Exsudat innerhalb des Absorptionskörpers (2) und damit innerhalb der Hülle (2) bis zur Entfernung des Absorptionskörpers von dem Körper des Patienten verbleibt, und wobei der Absorptionskörper sich in der Nähe seiner maximalen Füllung mit Flüssigkeit in seinem Querschnitt der Kreisform nähert,
**dadurch gekennzeichnet, dass**
der Einweg-Absorptionskörper(100) eine Menge an einer antimikrobiell wirkenden silberhaltigen Substanz aufweist, die in das Material des Textilabschnitts (1) oder der Hülle (2) eingebracht sind.

2. Einweg-Absorptionskörper(100) nach Anspruch 1, **dadurch gekennzeichnet, dass** die silberhaltige Substanz in Form eines in den Textilabschnitt (1) eingestreuten Granulates oder Pulvers vorliegt.

3. Einweg-Absorptionskörper(100) nach Anspruch 1 oder 2, **dadurch gekennzeichnet, dass** die silberhaltige Substanz (4) aus nanokristallinen atomaren oder elementaren silberhaltigen Verbindungen bestehen.

4. Einweg-Absorptionskörper(100) nach Anspruch 1 bis 3, **dadurch gekennzeichnet, dass** der Einweg-Absorptionskörper mit einem zusätzlichen, mit antimikrobiell wirkender, silberhaltiger Substanz ausgerüsteten, flüssigkeitsdurchlässigen Textilabschnitt (3) versehen ist, welcher innerhalb der Hülle (2) des Einweg-Absorptionskörpers angeordnet ist.

5. Einweg-Absorptionskörper(100) nach Anspruch 1 bis 3, **dadurch gekennzeichnet dass** eine weitere Lage (8) aus einer antiadhäsiven, glatten, mit Perforationen (9) versehenen Folie an der Außenfläche (5) wenigstens einer Seitenwand (2.1, 2.2) der Hülle (2) angeordnet ist, wobei an der Lage (8) trichterförmig nach Innen gerichtete Perforationen (9) eingearbeitet sind, wodurch eine verkleinerte Wundauflagefläche gebildet ist, und dass ein zusätzlicher mit antimikrobiell wirkender, silberhaltiger Substanz ausgerüsteter, flüssigkeitsdurchlässiger Textilabschnitt (3) zwischen der Hülle (2) und der weiteren Lage angeordnet ist.

6. Einweg-Absorptionskörper nach Anspruch 4, **dadurch gekennzeichnet, dass** der zusätzliche Textilabschnitt (3) von einer flüssigkeitsdurchlässigen Hülle (19) umgeben ist, die wiederum zwischen dem mit Superabsorber-Teilchen angereicherten Textilabschnittes (1), und einer der Seitenwände (2.1, 2.2) innerhalb der äußeren Hülle (2) liegt.

7. Einweg-Absorptionskörper nach Anspruch 1, **dadurch gekennzeichnet, dass** an der Außenfläche (5) wenigstens einer Seitenwand (2.1, 2.2) der Hülle (2) eine weitere Lage (17) einer flüssigkeitsdurchlässigen Folie von einer rauhen Oberflächenstruktur (11) angeordnet ist, die durch eine mechanische Reibung im Wundbereich die Granulation anregen kann.

8. Einweg-Absorptionskörper nach Anspruch 1 oder 2, **dadurch gekennzeichnet, dass** an der Außenfläche (5) einer der Seitenwände (2.1, 2.2), die im am Körper des Patienten angelegten Zustand dem Wundbereich abgewandt ist, eine Klebfolie in Form einer Hydrokolloid-Folie (10) angeordnet ist, deren Ausmaße größer als die der Hülle (2) sind.

9. Einweg-Absorptionskörper nach Anspruch 8, **dadurch gekennzeichnet, dass** die Hydrokolloid-Folie (10) in Form eines die Außenkante (7) überlappenden Ringes (18) auf die Hülle (2) angeklebt ist unter Belassung einer umlaufenden Klebefläche (13) zum Ankleben auf die Haut des Patienten.

10. Einweg-Absorptionskörper nach einem der vorhergehenden Ansprüche, **dadurch gekennzeichnet, dass** dieser durch einen flüssigkeitsundurchlässigen Folienabschnitt (14) bedeckt ist, dessen Ausmaße gröβer als die der Hülle (2) sind und an dessen Umfang eine Klebefläche (13) zum Ankleben des Absorptionskörpers auf die Haut des Patienten vorhanden ist, wobei der Folienabschnitt (14) wenigstens eine Anschlußstelle (12) zur Luftevakuierung aus dem Bereich der Wunde und zum Aufrechterhalten des Unterdrucks aufweist.

11. Einweg-Absorptionskörper nach Anspruch 5, **dadurch gekennzeichnet, dass** der Folienabschnitt (14) wenigstens teilweise mit der Hülle (2) verbunden ist.

12. Einweg-Absorptionskörper nach Anspruch 8, **dadurch gekennzeichnet, dass** der Folienabschnitt (14) an wenigstens einem Teil seiner Fläche transparent ist.

13. Einweg-Absorptionskörper nach einem der vorhergehenden Ansprüche, **dadurch gekennzeichnet, dass** der Textilabschnitt (1; 3) und/oder die Hülle (2) aus Mikro- bzw. Hydrofasern besteht.

14. Einweg-Absorptionskörper nach einem der vorhergehenden Ansprüche, **dadurch gekennzeichnet, dass** dieser Alginate aufweist.

15. Einweg-Absorptionskörper nach Anspruch 10, **dadurch gekennzeichnet, dass** mit Natrium oder Calcium versehene Alginat-Präparate aufweist.

## Claims

1. Disposable absorbent body (100) for absorbing liquid exudate issuing from a wound, having:
- a liquid-permeable cover (2) consisting of two side walls (2.1, 2.2),
- and at least one layer of a liquid-absorbing textile portion (1) containing superabsorber particles accommodated in the cover (2),
the absorbent exudate remaining within the absorbent body (2) and thus within the cover (2) until the absorbent body is removed from the patient's body, and the cross section of the absorbent body approximating a circular shape when it is almost filled with the maximum amount of liquid,
**characterized in that**
the disposable absorbent body (100) comprises an amount of a silver-containing substance which has an antimicrobial effect and is introduced into the material of the textile portion (1) or the cover (2).

2. Disposable absorbent body (100) according to Claim 1, **characterized in that** the silver-containing substance is in the form of a granular material or powder which is scattered into the textile portion (1).

3. Disposable absorbent body (100) according to Claim 1 or 2, **characterized in that** the silver-containing substance (4) consists of nanocrystalline atomic or elemental silver-containing compounds.

4. Disposable absorbent body (100) according to Claims 1 to 3, **characterized in that** the disposable absorbent body is provided with an additional liquid-permeable textile portion (3) which is finished with a silver-containing substance having an antimicrobial effect and is arranged within the cover (2) of the disposable absorbent body.

5. Disposable absorbent body (100) according to Claims 1 to 3, **characterized in that** a further layer (8) made up of an antiadhesive, smooth film provided with perforations (9) is arranged on the outer face (5) of at least one side wall (2.1, 2.2) of the cover (2), perforations (9), which are directed inwards in a funnel-shaped manner, being formed on the layer (8), thus forming a size-reduced wound dressing area, and **in that** an additional liquid-permeable textile portion (3) which is finished with a silver-containing substance having an antimicrobial effect, is arranged between the cover (2) and the further layer.

6. Disposable absorbent body according to Claim 4, **characterized in that** the additional textile portion (3) is surrounded by a liquid-permeable cover (19) which is, in turn, positioned within the outer cover (2) between the textile portion (1) enriched with superabsorber particles and one of the side walls (2.1, 2.2).

7. Disposable absorbent body according to Claim 1, **characterized in that** a further layer (17) of a liquid-permeable film of a rough surface structure (11), which can stimulate granulation by mechanical friction in the wound region, is arranged on the outer face (5) of at least one side wall (2.1, 2.2) of the cover (2).

8. Disposable absorbent body according to Claim 1 or 2, **characterized in that** an adhesive film in the form of a hydrocolloid film (10), the dimensions of which are larger than those of the cover (2), is arranged on the outer face (5) of one of the side walls (2.1, 2.2) that is remote from the wound region when applied to the patient's body.

9. Disposable absorbent body according to Claim 8, **characterized in that** the hydrocolloid film (10), in the form of a ring (18) overlapping the outer edge (7), is adhesively bonded onto the cover (2), leaving a peripheral adhesive surface (13) for adhesively bonding to the patient's skin.

10. Disposable absorbent body according to one of the preceding claims, **characterized in that** the absorbent body is covered by a liquid-impermeable film portion (14), the dimensions of which are larger than those of the cover (2) and at the circumference of which an adhesive surface (13) is present for adhesively bonding the absorbent body to the patient's skin, the film portion (14) having at least one connection point (12) for evacuating air from the region of the wound and for maintaining the reduced pressure.

11. Disposable absorbent body according to Claim 5, **characterized in that** the film portion (14) is at least partly connected to the cover (2).

12. Disposable absorbent body according to Claim 8, **characterized in that** the film portion (14) is transparent at at least a part of its surface.

13. Disposable absorbent body according to one of the preceding claims, **characterized in that** the textile portion (1; 3) and/or the cover (2) consists of microfibres or hydrofibres.

14. Disposable absorbent body according to one of the preceding claims, **characterized in that** the absorbent body comprises alginates.

15. Disposable absorbent body according to claim 10, **characterized in that** the absorbent body comprises alginate preparations provided with sodium or calcium.

## Revendications

1. Corps absorbant à usage unique (100) pour absorber un exsudat liquide sortant d'une plaie, comportant :
- une enveloppe (2) perméable au liquide et constituée de deux faces latérales (2.1, 2.2),
- et au moins une couche d'un morceau de textile (1), absorbant le liquide et contenant des particules de polymère superabsorbant, qui est placée dans l'enveloppe (2),
l'exsudat absorbé restant à l'intérieur du corps absorbant (100) et donc à l'intérieur de l'enveloppe (2) jusqu'à ce que le corps absorbant soit retiré du corps du patient et le corps absorbant ayant une section transversale proche de la forme circulaire lorsqu'il est pratiquement rempli au maximum de liquide,
**caractérisé en ce que** le corps absorbant à usage unique (100) comporte une certaine quantité de substance contenant de l'argent à action antimicrobienne qui est placée dans le matériau du morceau de textile (1) ou de l'enveloppe (2).

2. Corps absorbant à usage unique (100) selon la revendication 1, **caractérisé en ce que** la substance contenant de l'argent est présente sous forme de granulat ou de poudre dispersé dans le morceau de textile (1).

3. Corps absorbant à usage unique (100) selon la revendication 1 ou 2, **caractérisé en ce que** la substance (4) contenant de l'argent est constituée de combinaisons nanocristallines atomiques ou élémentaires contenant de l'argent.

4. Corps absorbant à usage unique (100) selon la revendication 1 à 3, **caractérisé en ce que** le corps absorbant à usage unique est muni d'un morceau de textile supplémentaire (3) qui est perméable au liquide et pourvu d'une substance contenant de l'argent à action antimicrobienne et qui est placé à l'intérieur de l'enveloppe (2) du corps absorbant à usage unique.

5. Corps absorbant à usage unique (100) selon la revendication 1 à 3, **caractérisé en ce qu'**une autre couche (8) constituée d'une feuille antiadhésive, lisse et munie de perforations (9) est agencée sur la surface extérieure (5) d'au moins une face latérale (2.1, 2.2) de l'enveloppe (2), des perforations en forme d'entonnoirs dirigés vers l'intérieur (9) étant pratiquées dans la couche (8), ce qui réduit la surface en contact avec la plaie, et **en ce qu'**un morceau de textile supplémentaire (3) perméable au liquide et pourvu d'une substance contenant de l'argent à action antimicrobienne est agencé entre l'enveloppe (2) et l'autre couche.

6. Corps absorbant à usage unique selon la revendication 4, **caractérisé en ce que** le morceau de textile supplémentaire (3) est entouré d'une enveloppe perméable au liquide (19) qui se trouve quant à elle entre le morceau de textile (1) enrichi en particules de polymère superabsorbant et l'une des faces latérales (2.1, 2.2) à l'intérieur de l'enveloppe extérieure (2).

7. Corps absorbant à usage unique selon la revendication 1, **caractérisé en ce qu'**une autre couche (17) d'une feuille perméable au liquide qui a une structure de surface (11) rugueuse et qui peut stimuler la granulation par un frottement mécanique dans la zone de la plaie est agencée sur la surface extérieure (5) d'au moins une face latérale (2.1, 2.2) de l'enveloppe (2).

8. Corps absorbant à usage unique selon la revendication 1 ou 2, **caractérisé en ce qu'**une feuille adhésive sous la forme d'une feuille hydrocolloïde (10) dont les dimensions sont supérieures à celles de l'enveloppe (2) est placée sur la surface extérieure (5) de celle des faces latérales (2.1, 2.2) qui est éloignée de la zone de la plaie lorsque le corps absorbant est appliqué sur le corps du patient.

9. Corps absorbant à usage unique selon la revendication 8, **caractérisé en ce que** la feuille hydrocolloïde (10) est collée sous la forme d'un anneau (18) chevauchant l'arête extérieure (7) tout en laissant une surface adhésive périphérique (13) pour le collage sur la peau du patient.

10. Corps absorbant à usage unique selon l'une des revendications précédentes, **caractérisé en ce que** celui-ci est couvert par un morceau de feuille (14) qui est imperméable au liquide, dont les dimensions sont supérieures à celles de l'enveloppe (2) et à la périphérie duquel une surface adhésive (13) est prévue pour le collage du corps absorbant sur la peau du patient, le morceau de feuille (14) comportant au moins une zone de liaison (12) pour l'évacuation de l'air de la zone de la plaie et pour le maintien de la dépression.

11. Corps absorbant à usage unique selon la revendication 5, **caractérisé en ce que** le morceau de feuille (14) est au moins partiellement relié à l'enveloppe (2).

12. Corps absorbant à usage unique selon la revendication 8, **caractérisé en ce que** le morceau de feuille (14) est transparent sur au moins une partie de sa surface.

13. Corps absorbant à usage unique selon l'une des revendications précédentes, **caractérisé en ce que** le morceau de textile (1 ; 3) et/ou l'enveloppe (2) sont en microfibres ou en hydrofibres.

14. Corps absorbant à usage unique selon l'une des revendications précédentes, **caractérisé en ce que** celui-ci comporte des alginates.

15. Corps absorbant à usage unique selon la revendication 10, **caractérisé en ce que** celui-ci comporte des préparations à l'alginate contenant du sodium ou du calcium.
